# EUROPÄISCHE PATENTSCHRIFT

(11) **EP 3 681 445 B1**
(45) Veröffentlichungstag und Bekanntmachung des Hinweises auf die Patenterteilung: **10.01.2024**
(21) Anmeldenummer: 18762070.3
(22) Anmeldetag: 28.08.2018
(51) Int. Cl.: A61F 5/01

(54) **GELENKEINRICHTUNG**
JOINT DEVICE
DISPOSITIF D'ARTICULATION

(30) Priorität: 14.09.2017 DE 102017121343
(43) Veröffentlichungstag der Anmeldung: 22.07.2020
(73) Patentinhaber: Otto Bock Healthcare Products GmbH, 1110 Wien (AT)
(72) Erfinder: SIMA, Harald, 3130 Herzogenburg (AT); AUBERGER, Roland, 1140 Wien (AT)
(74) Vertreter: Gramm, Lins & Partner Patent- und Rechtsanwälte PartGmbB
(86) Internationale Anmeldenummer: PCT/EP2018/073131
(87) Internationale Veröffentlichungsnummer: WO 2019/052805

(56) Entgegenhaltungen:
- EP-A1- 1 588 678
- DE-U1-202013 009 698
- US-A1- 2004 002 674
- US-A1- 2015 100 006
- US-A1- 2016 015 589

## Beschreibung

Die Erfindung betrifft eine Gelenkeinrichtung einer Orthese mit einem Oberteil und einem gelenkig daran angeordneten Unterteil, mit einer ersten Befestigungseinrichtung zur Festlegung des Oberteils an einem Patienten und einer zweiten Befestigungseinrichtung zur Festlegung des Unterteils an einer Gliedmaße eines Patienten, wobei die Gelenkeinrichtung das Oberteil mit dem Unterteil gelenkig verbindet und eine Oberteilanbindung sowie eine Unterteilanbindung aufweist, über die das Oberteil und das Unterteil an den Befestigungseinrichtungen festlegbar sind.

Orthesen werden an einen Patienten an dessen Rumpf und/oder Gliedmaße angelegt und weisen in der Regel Schienen oder Schalen mit Einrichtungen zum Festlegen der jeweiligen Schienen oder Schalen an dem Rumpf oder der Gliedmaße auf. Die Schienen oder Schalen sind über eine Gelenkeinrichtung miteinander verbunden, so dass eine gelenkübergreifende Anordnung der Orthese an dem Patienten stattfinden kann. Über eine Orthese können Bewegungen geführt, Verschwenkwinkel um eine Gelenkachse begrenzt, Verschwenkbewegungen verhindert oder eine Ausrichtung von Gliedmaßen zueinander unterstützt oder festgelegt werden. Darüber hinaus können Orthesen mit Dämpfer- und/oder Federelementen oder Antrieben versehen sein, um Verschwenkbewegungen um die jeweilige Gelenkachse zu dämpfen oder zu bewirken bzw. zu unterstützen. Der Dämpfer und/oder der Antrieb können mit einer Steuerung versehen sein, so dass in Abhängigkeit von Belastungen, Schwenkwinkeln, Geschwindigkeiten oder Beschleunigungen, die über Sensoren erfasst und in einer Steuerungseinrichtung ausgewertet werden, eine veränderte Dämpfung oder eine veränderte Bewegungsunterstützung in Flexionsrichtung oder Extensionsrichtung bereitgestellt werden kann.

Die Dämpfereinrichtungen können als hydraulische Dämpfereinrichtungen, pneumatische Dämpfereinrichtungen oder eine Kombination aus hydraulischen und pneumatischen Dämpfereinrichtungen ausgebildet sein. Ebenfalls können Dämpfer über magnetorheologische Stoffe durch Veränderung eines angelegten Magnetfeldes den Widerstand gegen eine Bewegung verändern. Neben rein passiven Dämpfereinrichtungen existieren Orthesen mit aktiven Antrieben, bei denen über beispielsweise einen Elektromotor eine Verlagerung des Oberteils relativ zu dem Unterteil in einer Verlagerungsrichtung bewirkt wird.

Orthesen können nur ein einziges Gelenk übergreifen, beispielsweise ein Hüftgelenk, ein Kniegelenk oder ein Knöchelgelenk, alternativ ist es möglich und vorgesehen, dass die Orthese mehrere Gelenke übergreift, beispielsweise sowohl das Hüftgelenk als auch das Kniegelenk und das Knöchelgelenk oder nur ein Hüftgelenk und ein Kniegelenk. Bei einer Orthese, die ein oder mehrere natürliche Gelenke übergreift, ist die jeweils proximale Komponente der Orthese das Oberteil und die jeweils distale Komponente das Unterteil. Bei einer Knie-Knöchel-Fuß-Orthese ist somit eine Unterschenkelschiene hinsichtlich des knieübergreifenden Abschnitts das Unterteil, da sich die Unterschenkelschiene distal zu einer Oberschenkelschiene befindet. Hinsichtlich einer Fußschiene oder Fußschale ist die Unterschenkelschiene dann ein Oberteil, während die Fußschiene oder Fußschale, die über ein Orthesen-Knöchelgelenk mit der Unterschenkelschiene verbunden ist, das Unterteil ausbildet. Bei einer das Hüftgelenk übergreifenden Orthese ist derjenige Teil, der am Rumpf oder Becken des Patienten angeordnet ist, das Oberteil, während das am Oberschenkel befestigte Element der Orthese das Unterteil bildet.

Bei der Anordnung von Aktuatoren, also Antrieben und/oder Dämpfern, an einer Orthese besteht das Problem, dass es aufgrund unterschiedlicher Anatomien, Fertigungstoleranzen und Weichteilverschiebungen im Bereich der Anlagestellen der Orthese an dem Körper des Patienten zu Fluchtungsfehlern zwischen der Orthesenachse und der anatomischen Gelenkachse kommen kann. Es kann insbesondere bei komplexeren Gelenken wie dem Hüftgelenk oder dem Schultergelenk, aber auch bei den Kniegelenk und Knöchelgelenk, schwierig sein, mit einer starren Konstruktion der Gelenkeinrichtung zwischen dem Oberteil und dem Unterteil eine ausreichende Kongruenz zwischen den anatomischen Achsen bzw. der anatomischen Achse und der Achse oder den Achsen der Orthese bereitzustellen.

Die US 2004/0002674 A1 betrifft eine anatomisch geformte Knieorthese mit medial und lateral angeordneten Gelenkeinrichtungen mit sphärisch geformten Gelenkkörpern mit darin ausgebildeten und angeordneten Schlitzen und Vorsprüngen, um eine sphärische Verschwenkung zusammen mit einer Zwangsverschiebung zu ermöglichen.

Die US 2016/0015589 A1 wird als nächstliegender Stand der Technik angesehen und betrifft eine Hüftorthese mit einem Antrieb, über den eine Oberschenkelschiene, die sich an einem Oberschenkel abstützt, in Flexionsrichtung und/oder Extensionsrichtung angetrieben werden kann. Die Oberschenkelschiene ist schwenkbar und längsverschieblich dazu an der Beckenhalterung angeordnet. Eine Oberschenkelhalterung ist drehbar an der Oberschenkelschiene gelagert.

Die DE 20 2013 009 698 U1 betrifft eine Parallelkinematik für eine Orthese oder ein Exoskelett mit Kopplungsstellen zu einer Extremität. Diese Kopplungsstellen sind seitlich zugänglich, weil Orthesengelenke für die Oberarmrotation und die Unterarmpronation und Unterarmsupination außerhalb der Extremität liegen. Die Kopplungsstellen sind an der Hand und dem Oberarm angeordnet, die Gelenke übergreifen das Handgelenk und das Ellenbogengelenk.

Die EP 1 588 678 A1 betrifft eine Hüftorthese zum Anlegen im Bereich der Hüfte mit einem Beckenring zur Festlegung an dem Becken und einem Kondylenteil zur Festlegung an dem Oberschenkel, die über eine Femur-Druckplatte und ein Kettengelenk miteinander verbunden sind. Das Kettengelenk kann aus mehreren Einzelgelenken bestehen, die hintereinander angeordnet sind.

Die US 2015/0100006 A1 betrifft eine winkeleinstellbare Rehabilitationsvorrichtung mit einer Oberschenkelschiene und einer Unterschenkelschiene, die gelenkig miteinander verbunden sind. Zwischen zwei seitlichen Abdeckungen der beiden Schienen im Bereich des Gelenkes ist ein Trennelement aus einem weicheren Material als die Schienen angeordnet, beispielsweise aus Plastik oder Gummi, um einerseits eine Schutzfunktion und andererseits eine Gleitfunktion bereitzustellen. Aufgabe der vorliegenden Erfindung ist es, eine Gelenkeinrichtung für eine Orthese bereitzustellen, mit der eine dem natürlichen Gelenk ortsnahe Positionierung der Gelenkeinrichtung und eine stets körpernahe Anlage der Orthese an dem Patienten möglich ist.

Erfindungsgemäß wird diese Aufgabe durch eine Gelenkeinrichtung mit den Merkmalen des Hauptanspruches gelöst. Vorteilhafte Ausgestaltungen und Weiterbildungen der Erfindung sind in den Unteransprüchen, der Beschreibung und den Figuren offenbart.

Die Gelenkeinrichtung einer Orthese mit einem Oberteil und einem gelenkig angeordneten Unterteil, mit einer ersten Befestigungseinrichtung zur Festlegung des Oberteils an einem Patienten und einer zweiten Befestigungseinrichtung zur Festlegung des Unterteils an einer Gliedmaße, wobei die Gelenkeinrichtung das Oberteil mit dem Unterteil gelenkig verbindet und eine Oberteilanbindung und eine Unterteilanbindung aufweist, über die das Oberteil und das Unterteil an den Befestigungseinrichtungen und der Gelenkeinrichtung festlegbar sind, sieht vor, dass die Gelenkeinrichtung mindestens vier Freiheitsgrade aufweist. Die Gelenkeinrichtung als solche ist aufgrund ihrer mindestens vier Freiheitsgrade kinematisch unbestimmt, so dass die Orthese als System, deren Bestandteil die Gelenkeinrichtung ist, erst durch das Anlegen an den Patienten oder an den Anwender kinematisch bestimmt wird. Die Gelenkeinrichtung als solche ist bevorzugt als ein vorgefertigtes Modul ausgebildet, das mit der Oberteilanbindung und der Unterteilanbindung an der jeweiligen Befestigungseinrichtung verbunden werden kann, beispielsweise verschraubt oder vernietet. Die jeweilige Befestigungseinrichtung kann als Schale, die den jeweiligen Körperteil zumindest teilweise umgreift, oder als Riemen oder Gurtanordnung ausgebildet sein, um insgesamt die Orthese an dem Patienten festzulegen. Bei einer das Hüftgelenk übergreifenden Orthese ist die erste Befestigungseinrichtung zur Festlegung des Oberteils an einem Patienten beispielsweise ein Beckengurt oder eine Beckenschale, während die Befestigungseinrichtung zur Festlegung des Unterteils als Oberschenkelschale oder Oberschenkelgurt ausgebildet sein kann. Bei der Anwendung als Schulterorthese ist das Oberteil an einer Befestigungseinrichtung festgelegt, die an dem Rumpf angelegt ist, während die Befestigungseinrichtung für das Unterteil an dem Oberarm angeordnet ist und als Schale oder Manschette ausgebildet sein kann, die über einen Gurt oder über einen Klettverschluss vollständig um den Oberarm herum angelegt werden kann.

Durch die Ausgestaltung der Gelenkeinrichtung mit mindestens vier Freiheitsgraden ist es möglich, die Gelenkeinrichtung so auszubilden, dass eine selbständige Positionierung der Komponenten zueinander innerhalb gewisser Bewegungsgrenzen ermöglicht wird, wodurch die jeweils resultierenden Drehachsen der Gelenkeinrichtung immer durch den physiologischen Drehpunkt des natürlichen Gelenkes verlaufen, wodurch Zwänge, Bewegungseinschränkungen oder aus Fluchtungsfehlern resultierenden Gelenksbelastungen vermieden werden. Somit ist es nicht mehr notwendig, den anatomischen Drehpunkt exakt zu bestimmen und die Orientierung der Drehachsen der Gelenkeinrichtung präzise auf den einmal gefundenen anatomischen Drehpunkt auszurichten, vielmehr richtet sich die Gelenkeinrichtung nach dem Anlegen der Orthese selbständig innerhalb der vorgegebenen Grenzen der Beweglichkeit korrekt zu dem physiologisch korrekten Drehpunkt aus. Durch die Ausgestaltung der Gelenkeinrichtung mit Oberteilanbindung und Unterteilanbindung ist es möglich, die Befestigungseinrichtungen von der Gelenkeinrichtung zu trennen, so dass das Anlegen einer Orthese erleichtert wird. Die Erfindung sieht vor, dass zumindest einem rotatorischen Freiheitsgrad ein Aktuator zugeordnet ist, der als Antrieb oder Dämpfer ausgebildet sein kann. Rein passive Dämpfer können hydraulische Dämpfer, pneumatische Dämpfer oder magnetorheologische Dämpfer sein, wobei auch Kombinationen der Wirkweisen möglich sind. Auch andere mechanische Bremsen können angeordnet sein. Darüber hinaus können aktive Aktuatoren in Gestalt von Antrieben, insbesondere motorischen Antrieben, vorgesehen sein, um Bewegungen zu unterstützen und Kräfte oder Momente zur Unterstützung der jeweils durch den Patienten ausgeführten Bewegung aufzubringen. Werden die aufgebrachten Kräfte der jeweils von dem Patienten eingeleiteten Bewegung entgegengerichtet, bildet der Antrieb eine aktive Widerstandseinrichtung aus und ist dann ebenfalls ein Dämpfer. Somit können Antriebe sowohl zur Unterstützung als auch als Dämpfer eingesetzt werden. Den Dämpfern können Sensoren zugeordnet sein, die an der Orthese oder an dem Patienten angeordnet sind. Über die Sensoren werden Informationen über wirksame Kräfte, Momente, Geschwindigkeiten, Beschleunigungen, Relativpositionen zueinander oder Stellungen von Gliedmaßen oder Orthesenkomponenten im Raum erfasst, einer Steuerungseinrichtung zugeleitet und ausgewertet. Die Steuerungseinrichtung kann auf Grundlage der ausgewerteten Sensordaten über eine Verstelleinrichtung, die motorisch angetrieben ist, einen passiven Dämpfer in seinen Eigenschaften verändern, beispielsweise eine Dämpfung erhöhen oder verringern. Bei Antrieben kann auf Grundlage der ausgewerteten Sensordaten eine entsprechende Aktivierung des jeweiligen Antriebes erfolgen. Um einen Antrieb der Orthese oder auch nur eine Verstellung einer Dämpfereinrichtung zu bewirken, ist zumindest ein Energiespeicher der Gelenkeinrichtung zugeordnet.

Der Aktuator, also der Dämpfer oder Antrieb, ist in oder an einer Halterung gelagert, die zwischen der Oberteilanbindung und Unterteilanbindung, insbesondere zwischen dem Oberteil und dem Unterteil, angeordnet ist, wobei die Halterung selbst mehrteilig ausgebildet sein kann und zumindest zwei Freiheitsgrade aufweist, um Zwängungen und Verkantungen zu vermeiden. Die beiden Freiheitsgrade sind bevorzugt rotatorische Freiheitsgrade, wobei die Drehachsen der beiden Freiheitsgrade senkrecht aufeinander stehen, um Bewegungen zueinander nicht zu beeinflussen.

Bevorzugt weist die Gelenkeinrichtung drei rotatorische Freiheitsgrade auf, um auch Kugelgelenke, wie das Hüftgelenk oder das Schultergelenk, überbrücken zu können und im Rahmen einer orthetischen Versorgung zu unterstützen.

Die Gelenkeinrichtung kann mindestens einen translatorischen Freiheitsgrad aufweisen, so dass eine Verschiebemöglichkeit in mindestens einer Richtung ermöglicht wird. Die Gelenkeinrichtung kann in einer Weiterbildung der Erfindung mindestens zwei translatorische Freiheitsgrade aufweisen, so dass eine Verschiebemöglichkeit in zwei unterschiedlichen Richtungen ermöglicht wird. Durch die Verschieblichkeit entlang zumindest eines translatorischen Freiheitsgrades kann verhindert werden, dass eine Befestigungseinrichtung bei einer Anordnung der jeweiligen Gelenkeinrichtung an dem Körperteil verlagert wird. Es kann beispielsweise bei einer Ausgestaltung der Gelenkeinrichtung als Teil einer das Hüftgelenk übergreifenden Orthese ein translatorischer Freiheitsgrad normal zu der Sagittalebene frei sein, jedoch durch Endanschläge beschränkt werden. Dieser translatorische Freiheitsgrad bewirkt bei angelegter Orthese eine Ausrichtung des funktionellen Drehpunktes der Gelenkeinheit mit dem anatomischen Hüftgelenk. Darüber hinaus kann ein translatorischer Freiheitsgrad normal zur Horizontalebene gegeben sein, so dass die Unterteilanbindung längsverschieblich entlang der Längserstreckung des Oberschenkels erfolgen kann.

Eine Variante der Erfindung sieht vor, dass die Drehachsen zumindest zweier rotatorischer Freiheitsgrade einander schneiden, so dass Drehungen um die eine Drehachse keine Auswirkungen auf Bewegungen um die andere Drehachse haben.

Um auch Gelenke unterstützen zu können, deren Drehachsen oder Drehpunkte innerhalb des Körpers liegen, ist es vorteilhaft, wenn die Schwenkachse zumindest eines rotatorischen Freiheitsgrades außerhalb der Gelenkeinrichtung liegt, so dass beispielsweise für ein Hüftgelenk auch eine Abduktion und Adduktion des Beines um eine Schwenkachse senkrecht zur Frontalebene ermöglicht werden kann. Bevorzugt ist hierzu in der Gelenkeinrichtung eine Langlochführung mit einem gekrümmten Langloch vorgesehen, dessen Kontur einen Kreisbahnabschnitt aufweist, wobei der Mittelpunkt der Kreisbahn außerhalb der mechanischen Komponenten der Gelenkeinrichtung liegt. Bevorzugt liegt der Mittelpunkt dieser Kurvenbahn in dem Drehpunkt oder angenähert an dem Drehpunkt des natürlichen Gelenkes, an dem die Gelenkeinrichtung angeordnet ist. Durch Verkürzung des Langloches, z.B. durch Einführen elastischer Anschlagelemente, kann der Bewegungsbereich in diesem Freiheitsgrad reduziert werden. Ebenfalls kann das Verhalten des Gelenkes in diesem rotatorischen oder translatorischen Freiheitsgrad durch Einführen von starren oder elastischen Bändern zwischen dem in den Langlöchern geführten Element und den Endpunkten der Langlöcher beeinflusst werden. Mit starren Bändern können Anschläge geschaffen werden, wobei die Anschlagsposition durch die Länge der Bänder festgelegt wird. Elastische Bänder ermöglichen die Umsetzung eines elastischen Verhaltens um diesen Freiheitsgrad, wobei die Neutralposition durch die Länge der Bänder, und die Steifigkeit durch die Elastizitätseigenschaften der Bänder bestimmt werden. Durch die parallele Verwendung mehrerer unterschiedlich langer bzw. steifer Bänder kann ein progressives Verhalten erreicht werden. Bei einer Orthese der unteren Extremität wäre dies das Hüftgelenk, bei einer Orthese der oberen Extremität wäre dies das Schultergelenk.

Zumindest einem Freiheitsgrad ist zumindest ein elastisches Pufferelement oder Federelement zugeordnet, um eine Stabilisierung der Gelenkeinrichtung hinsichtlich des betreffenden Freiheitsgrades zu erreichen. Darüber hinaus wird über das Feder- oder Pufferelement eine Begrenzung der Verlagerbarkeit um diesen Freiheitsgrad oder in diesem Freiheitsgrad erreicht. Darüber hinaus kann über das Feder- oder Pufferelement neben einer Begrenzung des Verlagerungsweges oder Verlagerungswinkels erreicht werden, dass die Bewegung in oder um diesen Freiheitsgrad mit einem Widerstand versehen ist, beispielsweise einem Reibungswiderstand, um die Gelenkeinrichtung und das Verhalten der Gelenkeinrichtung an die individuellen Bedürfnisse anpassen zu können.

Das Feder- oder Pufferelement oder die Feder- oder Pufferelemente eines rotatorischen Freiheitsgrades können durch parallel zur Drehachse angeordnete Elastomerelemente ausgebildet sein, so dass durch eine Veränderung der Länge, der bevorzugt in Strangform ausgebildeten Elastomerelemente, die Drehsteifigkeit einfach und stufenlos eingestellt werden kann. Darüber hinaus kann durch die Formgebung des Querschnittes der Feder- oder Pufferelemente das gewünschte elastische Verhalten eingestellt werden. So kann beispielsweise durch eine Querschnittszunahme in Verlagerungsrichtung ein progressives elastisches Verhalten eingestellt werden. Auch durch Materialpaarungen ist es möglich, die entsprechenden elastischen Eigenschaften einzustellen. Durch Verwendung sehr steifer Feder- oder Pufferelemente kann ein harter Anschlag realisiert werden. Die Neutralposition bei elastischem Verhalten bzw. die Anschlagspositionen können durch den Durchmesser der verwendeten Feder- oder Pufferelemente beeinflusst werden. Dies ist insbesondere bei Verwendung sehr steifer Elemente interessant, da dadurch der Freiheitsgrad in der durch die entsprechend gewählten Durchmesser der Feder- oder Pufferelemente in einer definierten Position gesperrt werden kann.

Zumindest ein Freiheitsgrad, insbesondere ein rotatorischer Freiheitsgrad, ist über eine Langlochführung oder eine Schienenführung ausgebildet, um auf eine einfache und sichere Art und Weise die Zuordnungen der Komponenten zueinander aufrecht zu erhalten. Innerhalb der Langlochführung kann ein auswechselbarer Zapfen geführt sein, so dass im Bereich der Langlochführung eine Trennung der Gelenkeinrichtung in mehrere Komponenten erfolgen kann, so dass eine leichte Montierbarkeit und Demontierbarkeit der Gelenkeinrichtung und damit auch ein leichtes Anlegen der Orthese erreicht werden kann. Durch die Anordnung der Trennstelle innerhalb der Langlochführung wird ein großer Wegebereich bereitgestellt, innerhalb dessen eine Montage erfolgen kann. Somit müssen die Komponenten der Gelenkeinrichtung zur Montage nicht exakt zueinander ausgerichtet werden.

Die Erfindung betrifft ebenfalls eine Orthese mit einem Oberteil und einem Unterteil, die über eine Gelenkeinrichtung, wie oben beschrieben, miteinander verbunden sind.

Nachfolgend werden Ausführungsbeispiele der Erfindung anhand der beigefügten Figuren näher erläutert. Es zeigen:
- Figur 1 -: eine schematische Gesamtansicht einer Orthese;
- Figur 2 -: eine perspektivische Ansicht einer Gelenkeinrichtung ohne Befestigungseinrichtung;
- Figur 3 -: eine Variante der Figur 2 mit einem veränderten Steuergerät;
- Figur 4 -: eine Seitenansicht der Ausführungsform gemäß Figur 2;
- Figur 5 -: eine Frontalansicht der Figur 4;
- Figur 6 -: eine Draufsicht gemäß Figur 5;
- Figur 7 -: eine Einzeldarstellung einer Unterteilanbindung,
- Figur 8 -: eine Schnittdarstellung durch eine Oberteilanbindung, sowie
- Figur 9 -: eine Variante der Figur 8.

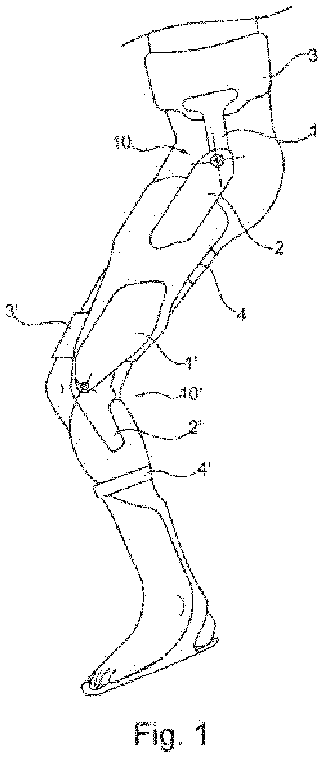

In der Figur 1 ist in einer schematischen Gesamtansicht eine Hüft-Knie-Knöchel-Orthese dargestellt, die drei Gelenke der unteren Extremität übergreift, nämlich das Hüftgelenk, das Kniegelenk und das Knöchelgelenk. In dem Bereich des natürlichen Hüftgelenkes und in dem Bereich des natürlichen Kniegelenkes ist jeweils eine Gelenkeinrichtung 10, 10'zwischen jeweils einem Oberteil 1, 1'und einem Unterteil 2, 2 angeordnet. Das jeweilige Oberteil 1, 1'ist über eine erste Befestigungseinrichtung 3, 3'an dem Patienten angelegt, das jeweilige Unterteil 2, 2'ist über eine zweite Befestigungseinrichtung 4, 4'an der jeweils distal zu der Gelenkeinrichtung 10, 10'befindlichen Gliedmaße festgelegt. Die Orthese weist somit zwei Oberteile 1, 1'auf. Das erste Oberteil 1 ist über einen Beckengürtel oder einen Hüftgürtel 3 an dem Rumpf des Patienten befestigt. Das dazugehörige Unterteil 2 ist über eine Oberschenkelschale oder Oberschenkelschiene und einen Befestigungsgurt als Befestigungseinrichtung 4 an dem Oberschenkel festgelegt. Die Oberschenkelschale zusammen mit der Befestigungseinrichtung 4 bildet gleichzeitig ein zweites Oberteil 1'aus, das über eine hierzu ersten Befestigungseinrichtung 3'an dem Oberschenkel festgelegt ist. Natürlich ist die Oberschenkelschale weiterhin mit der zu dem ersten Oberteil 1, zweiten Befestigungseinrichtung 4 weiterhin mit dem Oberschenkel verbunden. Distal zu der Oberschenkelschale oder Oberschenkelschiene ist eine Unterschenkelschiene als zweites Unterteil 2 angeordnet, das über einen Gurt als zweite Befestigungseinrichtung 4'an dem Unterschenkel festlegbar ist. Die Unterschenkelschiene weist eine Fußplatte auf, auf der der Fuß abgestützt wird. Die Oberschenkelschale mit den dazugehörigen Befestigungseinrichtungen ist somit in der Anwendung als mit zwei Gelenkeinrichtungen 10, 10'versehene Orthese sowohl erstes Unterteil 2 als auch zweites Oberteil 1'.

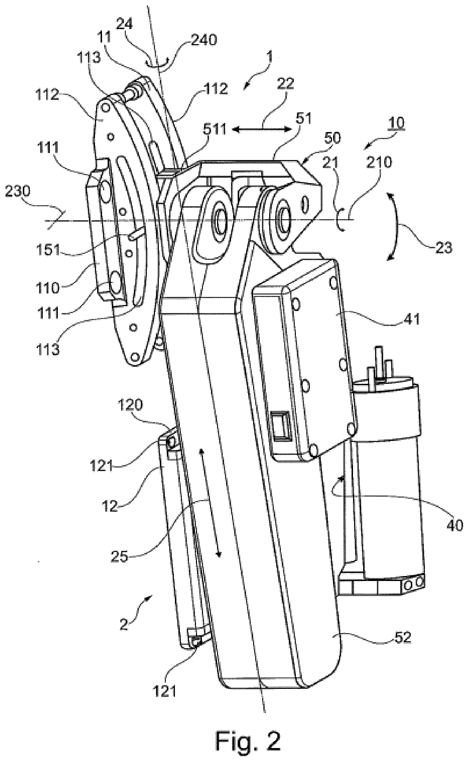

Figur 2 zeigt in einer perspektivischen Darstellung eine Gelenkeinrichtung 10 als Teil einer Orthese, die gelenkübergreifend an einem Patienten angeordnet werden kann. Die Gelenkeinrichtung 10 weist ein Oberteil 1 oberhalb einer ersten Drehachse 210 und ein Unterteil 2 auf, wobei das Oberteil 1 eine Oberteilanbindung 11 zur Festlegung an einer nicht dargestellten ersten Befestigungseinrichtung an dem Becken eines Patienten aufweist. Das Unterteil 2 weist eine Unterteilanbindung 12 auf, die an einer zweiten Befestigungseinrichtung zur Festlegung des Unterteils 2 an einem Oberschenkel befestigbar ist. Die Oberteilanbindung 11 ist mit einer Grundplatte 110 versehen, in der Bohrungen 111 angeordnet sind, über die die Oberteilanbindung 11 an der ersten Befestigungseinrichtung festlegbar ist. Die Festlegung kann beispielsweise über Schrauben erfolgen. Von der Grundplatte 110 erstrecken sich zwei Wände 112, wobei die beiden Wände 112 im Wesentlichen parallel zueinander orientiert und senkrecht zu der Grundplatte 110 ausgerichtet sind. Innerhalb jeder Wand 112 ist eine Langlochführung 113 ausgebildet, die als Teilkreisbogen oder zumindest gekrümmt ausgebildet ist, wobei der Radius der Langlochführung 113 so groß ist, dass der Mittelpunkt außerhalb der Oberteilanbindung 11 liegt.

Innerhalb der Langlochführung 113 ist ein Gleitstift 151 geführt, der die Oberteilanbindung 11 mit einer Halterung 50 verbindet, die mehrteilig ausgebildet ist. Der Gleitstift 151 verbindet die Halterung 50 mit der Oberteilanbindung 11 reversibel. Dazu ist der Gleitstift 151 in die Langlochführung 113 einführbar und ausführbar. Der Gleitstift 151 wird durch die Langlochführung 113 und durch eine Bohrung oder ein Langloch 5111 in einem Steg 511 hindurch geführt. Der Steg 511 ist Teil eines Rahmens 51, der wiederum Teil der Halterung 50 ist. Der Rahmen 51 ist im Wesentlichen U-förmig aufgebaut und bildet über seine Seitenschenkel eine Drehlagerung aus, so dass ein Gehäuse 52, das ein zweiter Teil der Halterung 50 ist, drehbar um die Drehachse 210 gelagert ist. Die Drehachse 210 steht im Wesentlichen senkrecht zur Sagittalebene und ermöglicht eine Schwenkbewegung des Unterteils 2 innerhalb der Sagittalebene, so dass bei einer an der Hüfte oder dem Becken angelegten Orthese ein Bein nach vorne und hinten schwingen kann.

Das Gehäuse 52 als Teil des Unterteils 2 dient zur Aufnahme eines Aktuators 40, der als Dämpfer und/oder Antrieb ausgebildet sein kann. Das Gehäuse 52 kann Teil eines Aktuatorgehäuses sein oder vollständig aus dem Aktuatorgehäuse bestehen. Lateral, also auf der der Oberteilanbindung 11 abgewandten Seite des Gehäuses 52, ist eine Steuerungseinrichtung 41 angeordnet, die mit nicht dargestellten Sensoren gekoppelt ist und durch Sensoren aufgenommene Sensordaten auswertet und einer Verstelleinrichtung oder einem Motorsteuergerät zuleitet, so dass entweder Dämpfereigenschaften verändert werden oder ein Antrieb aktiviert oder deaktiviert wird.

Distal zu der Oberteilanbindung 11 ist an der medialen, dem Patienten zugewandten Seite des Gehäuses 52 eine Unterteilanbindung 12 mit einer Grundplatte 120 und darin angeordneten Bohrungen 121 angeordnet. Über die Grundplatte 120 und die Bohrungen 121 wird die Unterteilanbindung 12 an der nicht dargestellten zweiten Befestigungseinrichtung festgelegt, so dass bei Einsatz der Gelenkeinrichtung als Hüftgelenk das Unterteil 2 an dem Oberschenkel des Patienten festgelegt werden kann.

Über die Gelenkeinrichtung 10 ist es möglich, den Rahmen 51 um eine Drehachse 230 zu verschwenken, da der Rahmen 51 innerhalb der Langlochführung 113 entlang eines Kreisbogenabschnitts geführt ist. Die Drehachse 230 liegt außerhalb der Gelenkeinrichtung 10. Bevorzugt ist der Radius der Langlochführungen 113 so gewählt, dass die Drehachse 230 innerhalb des Hüftgelenkes liegt, bevorzugt in dem anatomischen Hüftdrehpunkt.

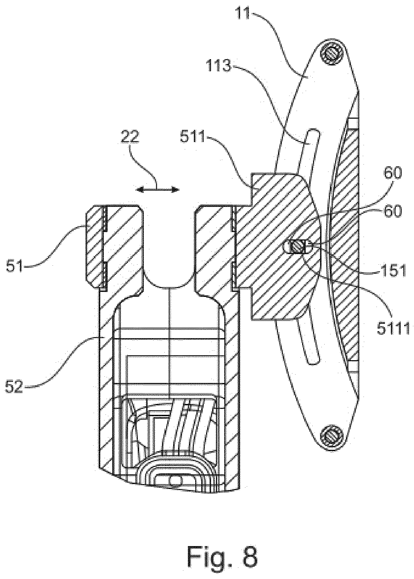

Das Gehäuse 52 und damit das gesamte Unterteil 2 ist an dem Rahmen 51 um die Drehachse 210 verschwenkbar gelagert. Darüber hinaus ist das Unterteil 2 in dem Rahmen 51 durch eine Langlochführung 5111 entlang der Drehachse 210 in Lateral- und Medialrichtung verschieblich angeordnet, was durch den Doppelpfeil 22, der einen translatorischen Freiheitsgrad repräsentiert, angedeutet ist. Die Verschieblichkeit entlang der Drehachse 210, also in Medial-Lateralrichtung, ist durch Endanschläge, zum Beispiel durch die in der Figur 8 dargestellten elastischen Pufferelemente 60 begrenzt, so dass nur eine eingeschränkte Verschieblichkeit innerhalb der Seitenschenkel des Rahmens 51 zugelassen ist. Alternativ zu einer Verschieblichkeit zwischen den Seitenschenkeln kann diese durch eine nachgiebige Lagerung des Steges 511 zwischen den Wänden 112 durch eine elastische Lagerung des Gleitstiftes 151 erfolgen. Alternativ kann die Unterteilanbindung 12 längsverschieblich entlang der Längserstreckung des Gehäuses 52, also in Proximal-Distal-Richtung des Unterschenkels an dem Gehäuse 52, gelagert werden, was durch den Doppelpfeil 25, der einen translatorischen Freiheitsgrad repräsentiert, angedeutet ist. Auch hier können Endanschläge die Verschieblichkeit der Unterschenkelanbindung 12 relativ zu dem Gehäuse 52 einschränken. Die Unterteilanbindung 12 ist darüber hinaus verschwenkbar um eine Drehachse 240, die in Proximal-Distal-Richtung, also in Längserstreckung des Gehäuses 52 verläuft, verschwenkbar gelagert, was weiter unten erklärt werden wird. Somit weist die Gelenkeinrichtung 1 insgesamt drei rotatorische Freiheitsgrade 21, 23 und 24 auf, die eine Verdrehbarkeit der Komponenten zueinander um die Drehachse 210, die Drehachse 230 und die Drehachse 240 zulassen. Darüber hinaus ist ein translatorischer Freiheitsgrad 22 innerhalb der Gelenkeinrichtung 1 vorhanden, nämlich in Medial-Lateral-Richtung durch die Verschieblichkeit innerhalb der Seitenschenkel des Rahmens 51.

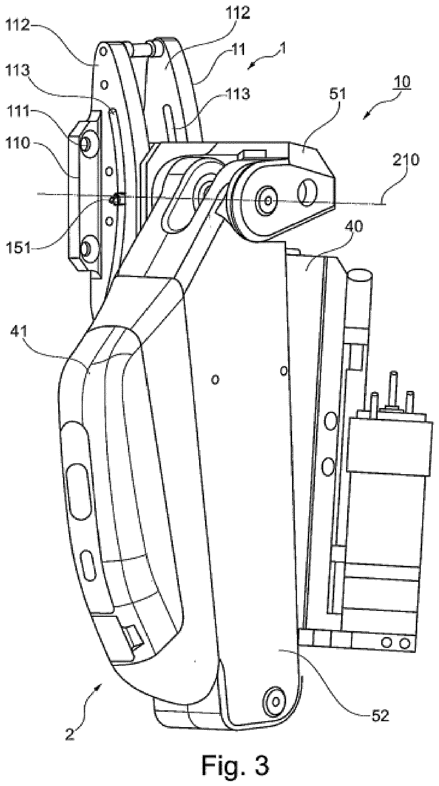

Figur 3 zeigt eine Variante der Gelenkeinrichtung 10 mit dem gleichen mechanischen Aufbau, jedoch mit einer frontal angeordneten Steuereinrichtung 41, durch die es möglich ist, die Gelenkeinrichtung 10 in Medial-Lateral-Richtung schmaler als bei der Variante gemäß Figur 2 auszubilden. In der Figur 3 ist zu erkennen, dass der Gleitstift 151, der den Rahmen 51 mit der Oberteilanbindung 11 verbindet, auf Höhe der Drehachse 210 liegt, so dass sich die Drehachsen 230 und 210 in einem Punkt schneiden.

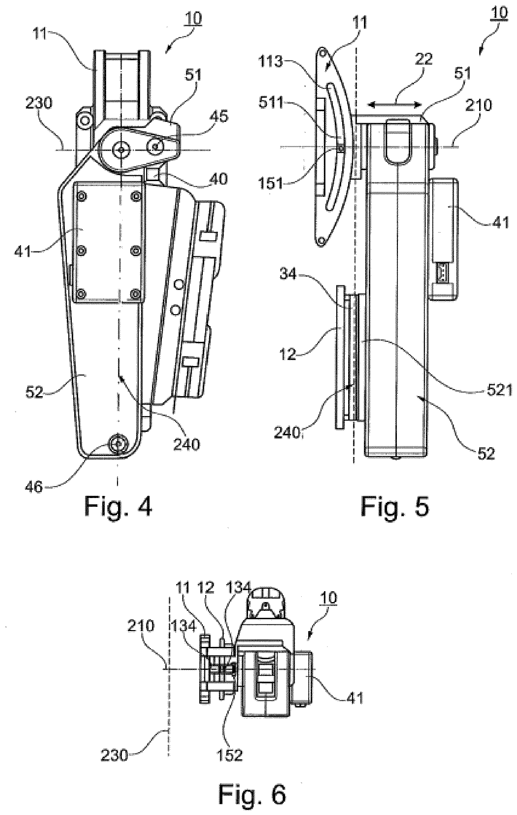

Figur 4 zeigt eine Seitenansicht der Gelenkeinrichtung 10 gemäß Figur 2, in der die Orientierung der Drehachsen 230 und 240 rechtwinklig zueinander zu erkennen ist. Ebenfalls ist der Aktuator 40 zu erkennen, der einen oberen Anlenkpunkt 45 beabstandet zu der Drehachse 210 aufweist. Ein unterer Anlenkpunkt 46 des Aktuators 40 in dem Gehäuse 52 des Unterteils 2 befindet sich in der dargestellten Ausführungsform fluchtend mit der Drehachse 240 der Unterteilanbindung 12. Durch eine Verschwenkung um die Drehachse 240 entlang des rotatorischen Freiheitsgrades 21 verändert sich der Abstand des oberen Anlenkpunktes 45 zu dem unteren Anlenkpunkt 46, so dass der Aktuator als beispielsweise Hydraulikdämpfer eine Relativbewegung zwischen Kolben und Zylinder in einer Longitudinalrichtung erfährt. Im Falle eines Dämpfers wird durch entsprechende hydraulische Widerstände ein Widerstand der Relativbewegung entgegengesetzt. Für einen Antrieb wird der Aktuator 40 extern mit Energie versorgt und eine Verlagerung der beiden Anlenkpunkte 45, 46 aufeinander zu oder voneinander weg motorisch bewirkt.

Figur 5 zeigt eine Frontalansicht der Gelenkeinrichtung 10 mit der lateral angeordneten Steuereinrichtung 41 und der medial zu dem Gehäuse 52 und dem Rahmen 51 angeordneten Oberteilanbindung 11. Der Steg 511 ist zwischen die Wände 112 der Oberteilanbindung 11 eingesetzt und wird durch den Gleitstift 151 gehalten. Der Rahmen 51 kann relativ zu der Oberteilanbindung 11 entlang des Freiheitsgrades 22 verschoben werden, alternativ dazu kann das Gehäuse 52 innerhalb des Rahmens 51 entlang der Drehachse 210 in Medial-Lateral-Richtung verschoben werden, um den translatorischen Freiheitsgrad 22 zu bilden.

Medial an dem Gehäuse 52 ist die Unterteilanbindung 12 um eine Drehachse 240 verschwenkbar angeordnet. Pufferelemente 34, die als strangförmige Elastomerelemente parallel zu der Drehachse 240 angeordnet sind, dienen einerseits als Endanschlag und andererseits als Rückstellelement, um neben der Ausbildung des rotatorischen Freiheitsgrades 24 um die Drehachse 240 eine Rückstellung in die dargestellte Ausgangsstellung zu bewirken. Über die geometrische Gestaltung der Pufferelemente 34, z.B. über deren Durchmesser, kann die Ausgangsstellung bzw. die Winkelposition der Endanschläge festgelegt werden. Zusätzlich kann das elastische Verhalten um diesen Freiheitsgrad durch die Geometrie und die Materialeigenschaften der Pufferelemente 34 beeinflusst werden.

Figur 6 zeigt eine Draufsicht auf die Gelenkeinrichtung gemäß Figur 5, in der zu erkennen ist, dass die Drehachse 230, um die eine Verschwenkung durch die Langlochführungen 113 möglich ist, lateral zu der Oberteilanbindung 11 und damit außerhalb der Gelenkeinrichtung 10 liegt. Die Drehachse 210 steht senkrecht auf der Drehachse 230.

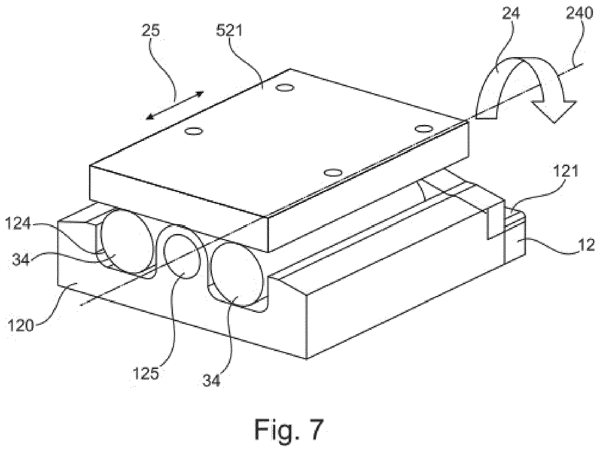

Figur 7 zeigt in einer Detailansicht die Unterteilanbindung 12 mit den Bohrungen 121 zur Festlegung an der nicht dargestellten Befestigungseinrichtung. Innerhalb der Grundplatte 120 sind Ausnehmungen 124 in Gestalt von Kanälen ausgebildet, zwischen denen eine Bohrung oder Hülse 125 angeordnet ist. Innerhalb der Bohrung 125 verläuft die Drehachse 240, um die die Unterteilanbindung 12 relativ zu einer Befestigungsplatte 521, die an dem Gehäuse 52 des Unterteils 2 über die dargestellten Bohrungen und Schrauben festlegbar ist, verschwenkt werden kann. Die Befestigungsplatte 521 ist auch in der Figur 5 zu erkennen.

Die Gelenkeinrichtung 10 ist durch die Ausgestaltung mit mindestens vier Freiheitsgraden kinematisch unbestimmt, das System der Orthese wird dadurch erst durch das Anlegen an den Anwender kinematisch bestimmt. In dem dargestellten Beispiel wird die Drehachse 210 normal zu der Sagittalebene selbständig durch das physiologische Gelenk, in diesem Fall das Hüftgelenk, positioniert. Trotz der Positionierung der Gelenkeinrichtung 10 außerhalb des Körpers verläuft die Drehachse 230 normal zu der Frontalebene durch den physiologischen Drehpunkt, was Zwänge und daraus resultierende Längsbelastungen vermeidet. Die exakte Bestimmung des anatomischen Drehpunktes des natürlichen Hüftgelenkes ist außerordentlich schwierig. Durch eine Gelenkeinrichtung 10, wie sie oben beschrieben ist, ist eine exakte Positionierung bei einer starren Anlegung der Orthese an dem menschlichen Körper nicht mehr erforderlich, da aufgrund der vorhandenen Freiheitsgrade eine selbständige Positionierung über den physiologischen Drehpunkt erfolgt.

Durch den herausführbaren Gleitstift 151 ist es möglich, die Gelenkeinrichtung 10 einfach montierbar und demontierbar auszubilden, um die Zuordnung der Komponenten der Gelenkeinrichtung 10 voneinander aufzuheben und wieder herzustellen. In dem Ausführungsbeispiel kann durch das Entfernen des Gleitstiftes 151 die Oberteilanbindung 11 leicht von den übrigen Komponenten der Gelenkeinrichtung 10 getrennt und wieder daran festgelegt werden, wodurch die Anlegung des Beckengurtes und eine separate Anlegung beispielsweise einer Oberschenkelschiene oder einer Oberschenkelschiene mit sich daran anschließender Unterschenkelschiene und einem Fußteil wesentlich erleichtert wird. Gerade bei einer Hüftgelenksanbindung mit sich daran anschließender Orthese, die das Kniegelenk und das Knöchelgelenk übergreift, ist anderenfalls eine Montage sehr schwer möglich, da sowohl das Knöchelgelenk als auch das Kniegelenk als auch das Hüftgelenk innerhalb der Orthese ausgerichtet und die Orthese an dem Bein festgelegt werden muss. Durch eine Auflösung der Zuordnung und einfache Wiederherstellbarkeit kann das Anlegen der Orthese wesentlich erleichtert werden.

Durch die Pufferelemente 34 wird die Gelenkeinrichtung 10 in ihrer Grundstellung stabilisiert. Passiv-elastische Eigenschaften können durch die Wahl der Geometrie und der Materialeigenschaften der Pufferelemente 34 festgelegt werden. Es ergibt sich eine insgesamt flache Bauhöhe, wobei die Unterteilanbindung 12 lateral versetzt zu der Oberteilanbindung 11 ausgebildet sein kann, um Entfernungsunterschiede in Medial-Lateral-Richtung des jeweiligen Patienten und bei der mechanischen Ausgestaltung des Oberteils 1 und des Unterteils 2 ausgleichen zu können. Die Gelenkeinrichtung 10 ermöglicht bei einer flachen Bauhöhe in Medial-Lateral-Richtung eine hohe Flexibilität hinsichtlich der Einstellbarkeit der gewünschten Neutralstellung und eine hohe Drehsteifigkeit. Die Neutralstellung und die Drehsteifigkeit können durch den Durchmesser, die Kontaktlänge und die elastischen Eigenschaften der jeweiligen Pufferelemente 34 festgelegt werden.

Die Veränderung der wirksamen Länge der Pufferelemente 34 kann konstruktiv durch eine entsprechende Formgebung und eine Verschiebemöglichkeit innerhalb der Kanäle 124 leicht realisiert werden, wodurch die Verdrehbarkeit und die Drehsteifigkeit um die Drehachse 240 leicht und stufenlos eingestellt werden kann.

Die Funktionalität der Hüftanbindung wird erreicht, indem die translatorischen Freiheitsgrade in der Sagittalebene gesperrt sind. Weiter ist der translatorische Freiheitsgrad normal zu der Sagittalebene grundsätzlich frei, jedoch durch Endanschläge beschränkt. Dieser zusätzliche Freiheitsgrad bewirkt bei angelegter Orthese eine Ausrichtung des funktionalen Drehpunktes der Gelenkeinrichtung 10 mit dem anatomischen Hüftdrehpunkt. Der rotatorische Freiheitsgrad in der Frontalebene ist passiv stabilisiert. Durch die Anpassung der rückstellenden, passiven Elemente oder Pufferelemente 34 kann die Steifigkeit für den jeweiligen Anwender optimal eingestellt werden. Die durch die Endanschläge 134 oder die Begrenzung des Langloches 5111 beschränkte Bewegungsmöglichkeit normal zur Sagittalebene ermöglicht eine Abweichung des physiologischen Drehpunktabstandes von dem durch die Gelenkeinrichtung 10 vorgegebenen Radius, der sich aus dem Radius der Langlochführung 113 ergibt. Dieses ermöglicht eine einfache Anpassung durch den Orthopädiemechaniker. Die Trennbarkeit der Oberteilanbindung von dem übrigen Teil der Gelenkeinrichtung 10 wird durch den Gleitstift 151 erreicht, der durch eine Stabilisierungsmutter 152 in dem Steg 511 geführt ist, an der elastische Endanschlagselemente 134 angreifen, die einer Verlagerung in Medial-Lateral-Richtung entgegenwirken.

Die Beinrotation wird durch die Pufferelemente 34 in ihrer Grundstellung stabilisiert und weist durch ihren Aufbau eine hohe Flexibilität hinsichtlich der gewünschten Drehsteifigkeit auf. Die Gelenkeinrichtung ist in ihrer Konstruktion so ausgeführt, dass die Rotation bei einem sehr flachen kompakten Aufbau ermöglicht wird, was sich sehr positiv auf die Medial-Lateral-Ausdehnung des Gesamtaufbaus der Orthese auswirkt.

In der Figur 8 ist eine Schnittdarstellung durch eine Oberteilanbindung 11 mit dem darin ausgebildeten, bogenförmigen Langloch 113, in dem der Steg 511 über den Gleitstift 151 geführt ist, gezeigt. An dem Steg 511 ist der Rahmen 51 und das nur teilweise dargestellte Gehäuse 52 befestigt und mit der Oberteilanbindung 11 gekoppelt. Innerhalb des Steges 511 ist ein Langloch 5111 ausgebildet, über das eine Medial-Lateral-Bewegung gemäß dem Doppelpfeil 22 ermöglicht wird. In dem Langloch 5111 können Endanschläge oder elastische Pufferelemente 60 angeordnet sein, um einerseits eine Ausrichtung in eine gewünschte Nulllage und andererseits eine elastische Verlagerbarkeit in Medial-Lateral-Richtung oder hinsichtlich des translatorischen Freiheitsgrades 22 zu ermöglichen.

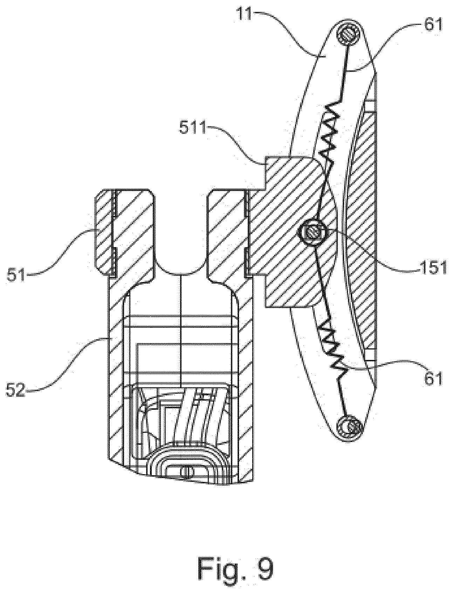

In der Figur 9 ist eine Variante der Figur 8 dargestellt, bei der dem Gleitstift 151 elastische Elemente 61 zugeordnet sind, die funktionell mit dem Gleitstift 151 verbunden sind. Dadurch wird sowohl der Gleitstift 151 als auch der Steg 511 elastisch in einer Nulllage innerhalb des Langloches 113 gehalten. Eine Verlagerung innerhalb des Langloches 113 ist weiterhin möglich. Die elastischen Elemente 61 können auch eine Begrenzung der Verlagerungsbewegung innerhalb des Langloches 113 darstellen, bevor die jeweiligen Enden des Langloches 113 erreicht werden. Statt elastischer Elemente 61 können auch starre Bänder, vorzugsweise mit einstellbarer Länge, dem Gleitstift 151 zugeordnet werden, um eine Anschlagsbegrenzung und eine Einstellung des Verschwenkwinkels zu gewährleisten.

## Patentansprüche

1. Gelenkeinrichtung einer Orthese mit einem Oberteil (1, 1') und einem gelenkig daran angeordneten Unterteil (2, 2'), mit einer ersten Befestigungseinrichtung (3, 3') zur Festlegung des Oberteils (1, 1') an einem Patienten und einer zweiten Befestigungseinrichtung (4, 4') zur Festlegung des Unterteils (2, 2') an einer Gliedmaße, wobei die Gelenkeinrichtung (10, 10') das Oberteil (1, 1') mit dem Unterteil (2, 2') gelenkig verbindet und eine Oberteilanbindung (11) und eine Unterteilanbindung (12) aufweist, über die das Oberteil (1, 1') und das Unterteil (2, 2') an den Befestigungseinrichtungen (3, 3', 4, 4') festlegbar sind, wobei die Gelenkeinrichtung (10, 10') mindestens vier Freiheitsgrade (21, 22, 23, 24, 25) aufweist, und wobei zumindest einem rotatorischen Freiheitsgrad (21) ein Aktuator (40) zugeordnet ist,
**dadurch gekennzeichnet, dass** der Aktuator (40) der in oder an einer Halterung (50) gelagert ist, die zwischen dem Oberteil (1, 1') und dem Unterteil (2, 2') angeordnet ist und die zumindest zwei Freiheitsgrade (21, 22, 24, 25) aufweist.

2. Gelenkeinrichtung nach Anspruch 1, **dadurch gekennzeichnet, dass** sie mindestens drei rotatorische Freiheitsgrade (21, 23, 24) aufweist.

3. Gelenkeinrichtung nach Anspruch 1 oder 2, **dadurch gekennzeichnet, dass** sie mindestens einen translatorischen Freiheitsgrad (22, 25) aufweist.

4. Gelenkeinrichtung nach einem der voranstehenden Ansprüche, **dadurch gekennzeichnet, dass** die Drehachsen (210, 230; 210, 240) zumindest zweier rotatorischer Freiheitsgrade (21, 23; 21, 24) einander schneiden.

5. Gelenkeinrichtung nach einem der voranstehenden Ansprüche, **dadurch gekennzeichnet, dass** die Schwenkachse (230) zumindest eines rotatorischen Freiheitsgrades (23) außerhalb der Gelenkeinrichtung (10, 10') liegt.

6. Gelenkeinrichtung nach einem der voranstehenden Ansprüche, **dadurch gekennzeichnet, dass** zumindest ein Freiheitsgrad (21, 22, 23, 24, 25) über Endanschläge (34, 134, 60, 61) begrenzt ist.

7. Gelenkeinrichtung nach einem der voranstehenden Ansprüche, **dadurch gekennzeichnet, dass** zumindest einem Freiheitsgrad (24) zumindest ein elastisches Pufferelement (34) zugeordnet ist.

8. Gelenkeinrichtung nach Anspruch 7, **dadurch gekennzeichnet, dass** das Pufferelement (34) eines rotatorischen Freiheitsgrades (24) durch parallel zur Drehachse angeordnete Elastomerelemente ausgebildet sind.

9. Gelenkeinrichtung nach Anspruch 7, **dadurch gekennzeichnet, dass** das Pufferelement (34) mit elastischen Zugelementen ausgebildet ist.

10. Gelenkeinrichtung nach Anspruch 7, **dadurch gekennzeichnet, dass** die Gelenkeinrichtung durch das zumindest eine Pufferelement (34) bezüglich zumindest eines Freiheitsgrades in einer Ausgangsstellung gehalten wird.

11. Gelenkeinrichtung nach einem der voranstehenden Ansprüche, **dadurch gekennzeichnet, dass** die beiden Freiheitsgrade (21, 24) der Halterung (50) rotatorische Freiheitsgrade sind und die Drehachsen (210, 240) senkrecht aufeinander stehen.

12. Gelenkeinrichtung nach einem der voranstehenden Ansprüche, **dadurch gekennzeichnet, dass** zumindest ein rotatorischer Freiheitsgrad (23) über eine Langlochführung (113) ausgebildet und/oder über Bänder (61) begrenzt ist.

13. Orthese mit einem Oberteil (1, 1') und einem Unterteil (2, 2'), die über eine Gelenkeinrichtung (10, 10') nach einem der voranstehenden Ansprüche miteinander verbunden sind.

## Claims

1. A joint device of an orthosis with an upper part (1, 1') and with a lower part (2, 2') arranged on the latter in an articulated manner, with a first fastening device (3, 3') for securing the upper part (1, 1') to a patient and a second fastening device (4, 4') for securing the lower part (2, 2') to a limb, wherein the joint device (10, 10') connects the upper part (1, 1') to the lower part (2, 2') in an articulated manner and has an upper-part connection (11) and a lower-part connection (12), via which the upper part (1, 1') and the lower part (2, 2') can be secured to the fastening devices (3, 3', 4, 4'), wherein the joint device (10, 10') has at least four degrees of freedom (21, 22, 23, 24, 25), and wherein an actuator (40) is assigned to at least one rotational degree of freedom (21), **characterized in that** the actuator (40) is mounted in or on a holder (50), which is arranged between the upper part (1, 1') and the lower part (2, 2') and has the at least two degrees of freedom (21, 22, 24, 25).

2. The joint device as claimed in claim 1, **characterized in that** it has at least three rotational degrees of freedom (21, 23, 24).

3. The joint device as claimed in claim 1 or 2, **characterized in that** it has at least one translational degree of freedom (22, 25).

4. The joint device as claimed in one of the preceding claims, **characterized in that** the rotation axes (210, 230; 210, 240) of at least two rotational degrees of freedom (21, 23; 21, 24) intersect each other.

5. The joint device as claimed in one of the preceding claims, **characterized in that** the pivot axis (230) of at least one rotational degree of freedom (23) lies outside the joint device (10, 10').

6. The joint device as claimed in one of the preceding claims, **characterized in that** at least one degree of freedom (21, 22, 23, 24, 25) is limited via end stops (34, 134, 60, 61).

7. The joint device as claimed in one of the preceding claims, **characterized in that** at least one elastic buffer element (34) is assigned to at least one degree of freedom (24).

8. The joint device as claimed in claim 7, **characterized in that** the buffer element (34) of a rotational degree of freedom (24) are formed by elastomer elements arranged parallel to the rotation axis.

9. The joint device as claimed in claim 7, **characterized in that** the buffer element (34) is formed with elastic tensioning elements.

10. The joint device as claimed in claim 7, **characterized in that** the joint device is held, by the at least one buffer element (34), in a starting position with respect to at least one degree of freedom.

11. The joint device as claimed in one of the preceding claims, **characterized in that** the two degrees of freedom (21, 24) of the holder (50) are rotational degrees of freedom, and the rotation axes (210, 240) are perpendicular to each other.

12. The joint device as claimed in one of the preceding claims, **characterized in that** at least one rotational degree of freedom (23) is formed via an elongate hole guide (113) and/or is limited via bands (61) .

13. An orthosis having an upper part (1, 1') and a lower part (2, 2'), which are connected to each other via a joint device (10, 10') as claimed in one of the preceding claims.

## Revendications

1. Dispositif d'articulation d'une orthèse comprenant une partie supérieure (1, 1') et une partie inférieure (2, 2') disposée de manière articulée sur celle-ci, comportant un premier dispositif de fixation (3, 3') destiné à immobiliser la partie supérieure (1, 1') sur un patient et un deuxième dispositif de fixation (4, 4') destiné à immobiliser la partie inférieure (2, 2') sur un membre, le dispositif d'articulation (10, 10') reliant de manière articulée la partie supérieure (1, 1') à la partie inférieure (2, 2') et comportant une attache de partie supérieure (11) et une attache de partie inférieure (12) qui permettent d'immobiliser la partie supérieure (1, 1') et la partie inférieure (2, 2') sur les dispositifs de fixation (3, 3', 4, 4'), le dispositif d'articulation (10, 10') présentant au moins quatre degrés de liberté (21, 22, 23, 24, 25), et un actionneur (40) étant associé à au moins un degré de liberté en rotation (21),
**caractérisé en ce que**
l'actionneur (40) est monté dans ou sur un support (50) qui est disposé entre la partie supérieure (1, 1') et la partie inférieure (2, 2') et qui présente au moins deux degrés de liberté (21, 22, 24, 25).

2. Dispositif d'articulation selon la revendication 1,
**caractérisé en ce qu'**il présente au moins trois degrés de liberté en rotation (21, 23, 24).

3. Dispositif d'articulation selon la revendication 1 ou 2,
**caractérisé en ce qu'**il présente au moins un degré de liberté en translation (22, 25).

4. Dispositif d'articulation selon l'une des revendications précédentes,
**caractérisé en ce que** les axes de rotation (210, 230 ; 210, 240) d'au moins deux degrés de liberté en rotation (21, 23 ; 21, 24) se croisent.

5. Dispositif d'articulation selon l'une des revendications précédentes,
**caractérisé en ce que** l'axe de pivotement (230) d'au moins un degré de liberté en rotation (23) est situé à l'extérieur du dispositif d'articulation (10, 10').

6. Dispositif d'articulation selon l'une des revendications précédentes,
**caractérisé en ce qu'**au moins un degré de liberté (21, 22, 23, 24, 25) est limité par des butées de fin de course (34, 134, 60, 61).

7. Dispositif d'articulation selon l'une des revendications précédentes,
**caractérisé en ce qu'**au moins un élément tampon élastique (34) est associé à au moins un degré de liberté (24).

8. Dispositif d'articulation selon la revendication 7,
**caractérisé en ce que** l'élément tampon (34) d'un degré de liberté en rotation (24) est réalisé par des éléments en élastomère disposés parallèlement à l'axe de rotation.

9. Dispositif d'articulation selon la revendication 7,
**caractérisé en ce que** l'élément tampon (34) est réalisé avec des éléments de traction élastiques.

10. Dispositif d'articulation selon la revendication 7,
**caractérisé en ce que** le dispositif d'articulation est maintenu dans une position initiale par rapport à au moins un degré de liberté par ledit au moins un élément tampon (34).

11. Dispositif d'articulation selon l'une des revendications précédentes,
**caractérisé en ce que** les deux degrés de liberté (21, 24) du support (50) sont des degrés de liberté en rotation, et les axes de rotation (210, 240) sont perpendiculaires l'un à l'autre.

12. Dispositif d'articulation selon l'une des revendications précédentes,
**caractérisé en ce qu'**au moins un degré de liberté en rotation (23) est réalisé par un guidage à trou oblong (113) et/ou est limité par des rubans (61).

13. Orthèse comprenant une partie supérieure (1, 1') et une partie inférieure (2, 2') qui sont reliées entre elles par un dispositif d'articulation (10, 10') selon l'une des revendications précédentes.
